# EUROPEAN PATENT APPLICATION

(11) **EP 2 077 146 A2**
(43) Date of publication of application: **08.07.2009**
(21) Application number: 08163804.1
(22) Date of filing: 05.09.2008
(51) Int. Cl.: B01D 53/52

(54) **Method and device for removal of hydrogen sulfide from biogas**

(30) Priority: 29.11.2007 PL 38391307
(71) Applicant: Politechnika Lubelska, 20-219 Lublin (PL)
(72) Inventor: Pawlowska, Malgorzata, 20-027, Lublin (PL); Pawlowski, Lucjan, 20-027, Lublin (PL)
(74) Representative: Kaminski, Piotr

(57) **Abstract**

Invention relates to a method and device for removal of hydrogen sulphide from biogas. A method is characterised in that the biogas is introduced to the device through a pipe and it flows out in its upper part and then flows around convex, non-permeable cover and is forced into a chamber in the shape of funnel with porous plates where there is an absorbing solution and while passing through the absorbing solution and porous plates, insoluble copper sulphide is formed, which following crystalisation settles on the funnel-shaped bottom of the chamber, while biogas devoid of hydrogen sulphide is collected from the device through outlet port. A device is characterised in that it consists of a chamber (8), which in its lower part forms a conical funnel (9) which ends with the outlet port (6) for discharge of sediment while in upper part of funnel (9) inside the chamber (8) there is a convex, non-permeable cover (2) with horizontal plates (4) and in the central part of chamber (8) from down there is a pipe (1) introducing the biogas to the chamber where there is an absorbing solution (3) and in the upper part of chamber (8) there is an outlet port (5) and in its side part there is an inlet port (7).

## Description

The present invention relates to a method and device for removal of hydrogen sulphide from biogas.

Methane, as well as carbon dioxide, is a gas responsible for greenhouse effect. Its source could be natural processes e.g. methane fermentation occurring in the swamp areas and methane production in the fields during rice cultivation.

Also, significant source of methane are ruminants, which during processes of feed digestion produce certain amounts of methane. Major source of methane can be waste disposal depots, where methane can be recuperated in the form of biogas and exploited for energy purposes. Yet when the methane contents in biogas drops below 30% its exploitation for energy purposes becomes unprofitable. Emission of biogas containing less then 30 % methane lasts tens of years which makes significant contribution to the greenhouse effect. Therefore research concerning removal of methane from biogas emitted by waste disposal depots is carried out. One of the methods for methane removal exploits bio-filters. Biogas is introduced into the bed made from porous material which creates good conditions for growth of microorganisms. The bed must have continuous access to air. Aeration intensifies the process of purifying of biogas from methane.

Depending on the type of waste disposal depot, its sealing system as well as its degassing, it is possible to use methanotrophic bed in the form of biofilter built into the layer covering the cap of the waste disposal depot, the so-called bio-windows. It is also possible to place the biofilter outside of this layer. In the former case, biogas would migrate through the layer of waste, and being under certain pressure, while meeting the porous material, it would pass through it purifying itself from bio-degradable components. In the latter case, where the biofilter is placed outside the layer, the biogas would be delivered to biofilter from the degassing system. It is also possible, that all the surface of the waste disposal depot is covered by the material of the bed, in which case it would be placed directly on a layer of waste. Today however, in view of applicable laws, such solution is not put in practice. Such application is possible only in case of old waste disposal depots, where the amounts of biogas emitted to atmosphere are little.

In the process of biofiltration of biogas one exploits natural abilities of microorgranisms to oxidate methane in aerobic conditions. In such process methane is oxidised to carbon dioxide. The second product of this reaction is water:

CH₄ + 2 O₂ → CO₂ + 2H₂O

Methane oxidising microorganisms (methanotrophs) are mainly bacteria, which are widespread in soil and water environments. They belong to microaerophilic aerobes (they prefer lower than atmospheric oxygen concentration). They have specific requirements as to pH and humidity of substrate, availability of elements and mineral salts, temperature and surface necessary for growth. They are sensitive to certain chemical compounds, e.g. ammonia, pesticides, hydrogen sulphide, presence of which causes decrease in their methanotrophic activity. During waste decomposition processes certain amounts of hydrogen sulphide are generated, which disturbs biochemical oxidation of methane to CO₂ occurring in biofilters. To improve efficiency of biochemical oxidation of methane, hydrogen sulphide has to be removed from biogas directed to biofilter.

In 2007 in journal "Waste Management" the attempts of removal of hydrogen sulphide were described. The used sorbent was obtained through pyrolysis of mixture of sludge and smoke-box dusts which was carried out at 950 °C. Drawback of this method was a complexity of the process of sorbent production as well as relatively large amounts of secondary waste product i.e. the sorbent with absorbed hydrogen sulphide.

In German patent nr 102006050610 a method for removal of hydrogen sulphide was suggested, whereby in pressurised scrubber, by use of acidic solution in temperature of 15-40 °C, H₂S is oxidised to S by atmospheric oxygen.

In Chinese patent nr 2870960 hydrogen sulphide is oxidised biologically in two towers where a layer of microorganisms is present.

In work published in Bioresource Technology, 2006 (p. 518-524), F.P Van Der Zee *et al*. propose the use of anaerobic reactor - with small amount of oxygene, in which H₂S contained in biogas is microbiologically oxidised at low flow (amounting to 0,7-0,9 m³ per day) while ensuring that molar ratio of oxygen to sulphur is in range 8-10. The drawback of this method is low once-through rate.

German patent 102004055162 describes a method for removal of hydrogen sulphide. First hydrogen sulphide coming from biogas is washed in water in which it dissolves. Water containing dissolved H₂S is then directed to the oxygen aerated zone, where hydrogen sulphide is oxidised to free sulphur.

Similarly Chinese patent 1724635 describes a device for removal of hydrogen sulphide and other organic compounds in water filter. Aqueous solution containing H₂S is directed to a container in which it is desulphurised and afterwards directed to a scrubber in order to remove CO_{2.} Following expelling of CO₂ in the scrubber, the water is recirculated to the process. The shortcoming of this method stems from low solubility of H₂S in water which calls for necessity of use of large quantities of water. Furthermore, during aeration certain small amounts of are released to the environment, which, due to high toxicity of hydrogen sulphide, necessitates its removal, which makes the process considerably more complex.

The disadvantage of all so far described methods is an incomplete removal of hydrogen sulphide from biogas, as a rule not exceeding 90%.

The essence of the method for removal of hydrogen sulphide from biogas is that the biogas is introduced to the device through a pipe, from which it flows out in its upper part and then flows around convex, non-permeable cover to enter a chamber in the shape of funnel having porous plates and containing absorbing solution with which biogas comes in contact and forms insoluble copper sulphide, which following crystalisation settles on the funnel-shaped bottom of the chamber, while biogas devoid of hydrogen sulphide is collected from the device through outlet port. Periodically the copper sulphide suspension is collected through funnel-shaped outlet port situated in the bottom of the chamber while at the same time the suspension of copper hydroxide is introduced through a port situated in the upper, side part of the chamber. CuSO₄ solution of molar concentration of 1 is the absorbing solution.

The essence of device for removal of hydrogen sulphide is that it consists of a chamber, which in its lower part forms a conical funnel which ends with the outlet port for discharge of sediment and in its upper part there is a convex, non-permeable cover having horizontal porous plates, while in the middle part of the chamber, form down, there is a pipe introducing the biogas in the chamber and in its upper part there is an outlet port and in its side part there is an inlet port.

Advantageous effect of this invention is that one obtains the sediment of copper sulphide, which is starting material in copper production. Device according to present invention is simple and functional in its performance.

### Example

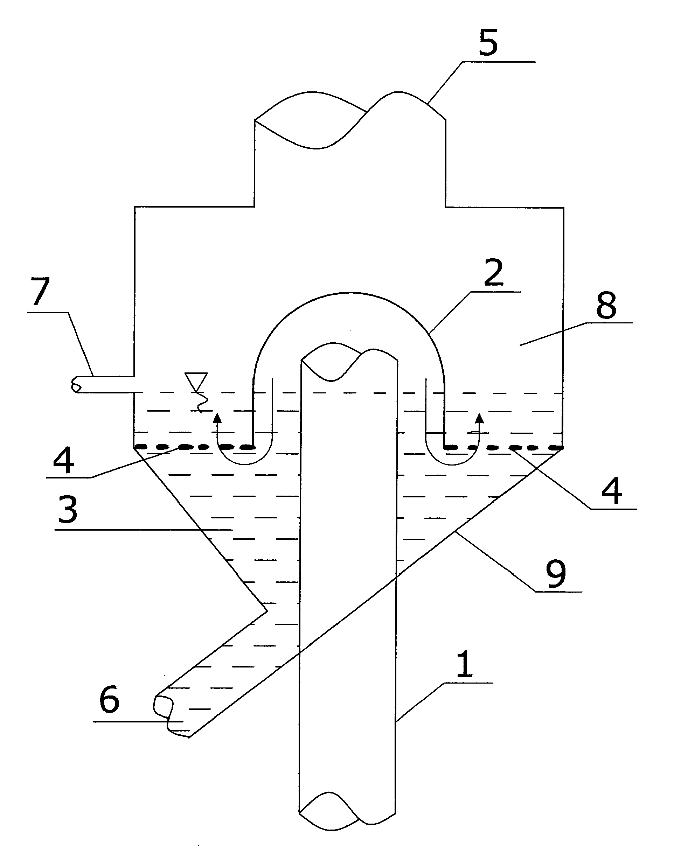

Biogas containing 150 mg of H₂S per 1 dm³ was transmitted at the speed of 10 m³/h through the device according to present invention, as depicted on the drawing. Every 24 hours a fraction of 1 dm³ was collected from the funnel. Following filtration and drying the collected fraction contained 179 g of CuS. 110 g of Ca(OH)₂ was added to the solution obtained after filtration, which had volume of 920 cm³. Then the solution was supplemented with distilled water to obtain the volume of 1 dm³. The suspension of copper hydroxide was introduced to the fennel through a port.

The device for removal of hydrogen sulphide consists of a chamber 8, which in its lower part forms a conical funnel 9 which ends with the outlet port 6 for discharge of sediment. Funnel 9 in its upper part is covered by a convex, non-permeable cover 2 which has horizontal plates 4 which are porous to allow for gas permeability. Concentrically situated in the funnel there is a pipe 1 for introducing the biogas. In the chamber 8 there is solution 3 which absorbs hydrogen sulphide (e.g. solution of copper sulphate) and forms sparingly soluble and easily crystallisable sediment.

The device operates as follows: the biogas is introduced to the chamber 8 through a pipe 1, from which it flows out in its upper part and then meets convex, non-permeable cover 2 and is forced into absorbing solution 3. It passes through it and through porous plates 4, at all times remaining in contact with the solution. Devoid of hydrogen sulphide it leaves chamber 8 through outlet port 5, situated in the upper part of chamber 8. The absorbing solution is the solution of CuSO₄ of molar concentration of 1 which reacts with H₂S and creates sparingly soluble copper sulphide, which following crystalisation settles on the bottom of the funnel 9. Periodically the copper sulphide suspension is collected through outlet port 6 while at the same time the suspension of copper hydroxide is introduced through a port 7.

## Claims

1. A method for removal of hydrogen sulphide from biogas **characterised in that** the biogas is introduced to the device through a pipe and it flows out in its upper part and then flows around convex, non-permeable cover and is forced into a chamber in the shape of funnel with porous plates where there is absorbing solution and while passing through the absorbing solution and porous plates, insoluble copper sulphide is formed, which following crystalisation settles on the funnel-shaped bottom of the chamber, while biogas devoid of hydrogen sulphide is collected from the device through outlet port.

2. A method according to claim 1 **characterised in that** periodically the copper sulphide suspension is collected through funnel-shaped outlet port situated in the bottom of the chamber while at the same time the suspension of copper hydroxide is introduced through a port situated in the upper, side part of the chamber.

3. A device for removal of hydrogen sulphide **characterised in that** it consists of a chamber (8), which in its lower part forms a conical funnel (9) which ends with the outlet port (6) for discharge of sediment while in upper part of funnel (9) inside the chamber (8) there is a convex, non-permeable cover (2) with horizontal plates (4) and in the central part of chamber (8), from down, there is a pipe (1) introducing the biogas to the chamber where there is an absorbing solution (3) and in the upper part of chamber (8) there is an outlet port (5) and in its side part there is an inlet port (7).
